# EUROPEAN PATENT APPLICATION

(11) **EP 2 263 551 A1**
(43) Date of publication of application: **22.12.2010**
(21) Application number: 10251101.1
(22) Date of filing: 16.06.2010
(51) Int. Cl.: A61B 10/02, A61B 17/34

(54) **MRI biopsy targeting grid wall guide**

(30) Priority: 17.06.2009 US 486196
(71) Applicant: Ethicon Endo-Surgery, Inc., Cincinnati, OH 45242 (US)
(72) Inventor: Mollere, Rebecca J., Loveland Ohio 45140 (US)
(74) Representative: Tunstall, Christopher Stephen

(57) **Abstract**

A targeting guide device (104,300) is configured to support a biopsy device (14). The guide device comprises a first plate (200,312) and a second plate (202,314). A biopsy device may be supported by either the first plate or the second plate. The targeting guide device is also configured to be secured to a compression grid plate (96), which is situated adjacent the tissue to be biopsied. The compression grid plate includes apertures (130) that are defined by grid walls (132,134). Either or both of the first plate and the second plate have a slot (204,206) within which a grid wall may be positioned so as to secure placement of the targeting guide device. In addition, either or both of the first plate and the second plate have an access hole (214,304) through which a biopsy device may be inserted. A flanged insert (302) may be positioned through the access hole to provide further support for the biopsy device.

## Description

### BACKGROUND

Biopsy samples have been obtained in a variety of ways in various medical procedures using a variety of devices. Biopsy devices may be used under stereotactic guidance, ultrasound guidance, MRI guidance, PEM guidance, BSGI guidance, or otherwise. Merely exemplary biopsy devices are disclosed in U.S. Pat. No. 6,273,862, entitled "Surgical Device for the Collection of Soft Tissue," issued Aug. 14, 2001; U.S. Pat. No. 6,231,522, entitled "Biopsy Instrument with Breakable Sample Segments," issued May 15, 2001; U.S. Pat. No. 6,228,055, entitled "Devices for Marking and Defining Particular Locations in Body Tissue," issued May 8, 2001; U.S. Pat. No. 6,120,462, entitled "Control Method for an Automated Surgical Biopsy Device," issued September 19, 2000; U.S. Pat. No. 6,086,544, entitled "Control Apparatus for an Automated Surgical Biopsy Device," issued July 11, 2000; U.S. Pat. No. 6,077,230, entitled "Biopsy Instrument with Removable Extractor," issued June 20, 2000; U.S. Pat. No. 6,017,316, entitled "Vacuum Control System and Method for Automated Biopsy Device," issued Jan. 25, 2000; U.S. Pat. No. 6,007,497, entitled "Surgical Biopsy Device," issued Dec. 28, 1999; U.S. Pat. No. 5,980,469, entitled "Method and Apparatus for Automated Biopsy and Collection of Soft Tissue," issued Nov. 9, 1999; U.S. Pat. No. 5,964,716, entitled "Method of Use for a Multi-Port Biopsy Instrument," issued Oct. 12, 1999; U.S. Pat. No. 5,928,164, entitled "Apparatus for Automated Biopsy and Collection of Soft Tissue," issued July 27, 1999; U.S. Pat. No. 5,775,333, entitled "Apparatus for Automated Biopsy and Collection of Soft Tissue," issued July 7, 1998; U.S. Pat. No. 5,769,086, entitled "Control System and Method for Automated Biopsy Device," issued June 23, 1998; U.S. Pat. No. 5,649,547, entitled "Methods and Devices for Automated Biopsy and Collection of Soft Tissue," issued July 22, 1997; U.S. Pat. No. 5,526,822, entitled "Method and Apparatus for Automated Biopsy and Collection of Soft Tissue," issued June 18, 1996; U.S. Pub. No. 2008/0214955, entitled "Presentation of Biopsy Sample by Biopsy Device," published September 4, 2008; U.S. Pub. No. 2007/0255168, entitled "Grid and Rotatable Cube Guide Localization Fixture for Biopsy Device," published November 1, 2007; U.S. Pub. No. 2007/0118048, entitled "Remote Thumbwheel for a Surgical Biopsy Device," published May 24, 2007; U.S. Pub. No. 2005/0283069, entitled "MRI Biopsy Device Localization Fixture," published December 22, 2005; U.S. Pub. No. 2003/0199753, entitled "MRI Compatible Biopsy Device with Detachable Probe," published Oct. 23, 2003; U.S. Pub. No. 2003/0109803, entitled "MRI Compatible Surgical Biopsy Device," published June 12, 2003; U.S. Provisional Patent Application Serial No. 60/874,792, entitled "Biopsy Sample Storage," filed December 13, 2006; and U.S. Provisional Patent Application Serial No. 60/869,736, entitled "Biopsy System," filed December 13, 2006. The disclosure of each of the above-cited U.S. Patents, U.S. Patent Application Publications, and U.S. Provisional Patent Applications is incorporated by reference herein.

Some biopsy systems may provide an apparatus to guide a probe and/or other components of a biopsy device to a desired biopsy site. In some such biopsy systems, a targeting bracket and positioning grid plate may be used. The targeting bracket may be selectively engaged with an opening in the grid plate. The targeting bracket may include guide holes to receive a portion of the probe and/or other components, for example a needle, cannula, obturator, or combinations of these or other components. With the targeting bracket engaged with the grid plate, the probe or other components can be guided through a selected guide hole of the targeting bracket to arrive at a desired biopsy site. The desired biopsy site may or may not have been identified and/or targeted by one or more of the guidance approaches mentioned above. In some situations, it might be desirable to provide a targeting bracket with features that improve a targeting bracket's use with one or more positioning grid plates.

While several systems and methods have been made and used for obtaining a biopsy sample, it is believed that no one prior to the inventors has made or used the invention described in the appended claims.

The following is a non-exhaustive list of embodiments of the invention that are or may be claimed.
1. An apparatus for guiding a biopsy instrument into a breast of a patient supported upon a patient support surface, the apparatus comprising:
   (a) a pair of compressive members movably attached to the patient support surface on opposing sides to locate the patient's breast, a selected one of the pair of compressive members further comprising a grid plate having a plurality of grid apertures defined by a plurality of grid walls, the grid plate being accessible from a lateral side and being at least substantially open from a medial side presented to the breast; and
   (b) a bracket comprising a first plate and a second plate, wherein the first plate has a slot defined by two side surfaces, wherein the second plate defines a guide opening configured to permit the insertion of a portion of the biopsy instrument through the second plate;
   wherein the slot of the first plate is engaged with a grid wall of the grid plate, wherein the engagement of the grid wall with the slot of the first plate allows the insertion of the biopsy instrument through the guide opening of the second plate and a grid aperture.
2. The apparatus of embodiment 1, wherein the first plate and the second plate are at a substantially right angle relative to each other.
3. The apparatus of embodiment 1, further comprising an insert member disposed in the guide opening of the second plate.
4. The apparatus of embodiment 3, wherein the insert member comprises a flange and a longitudinally extending hollow shaft configured to insertingly receive a portion of the biopsy instrument.
5. The apparatus of embodiment 1, wherein the second plate has a slot defined by two side surfaces, the slot being configured to receive a selected one of the grid walls.
6. The apparatus of embodiment 1, wherein the first plate has at least one guide opening configured to permit the insertion of the biopsy instrument through the first plate.
7. The apparatus of embodiment 1, wherein the two side surfaces of the slot are substantially parallel to each other.
8. The apparatus of embodiment 1, wherein the second plate defines a plurality of guide openings.
9. The apparatus of embodiment 1, wherein the bracket it secured to the grid plate by friction between the two side surfaces of the slot and the grid wall.
10. The apparatus of embodiment 9, wherein the two side surfaces defining the slot include an elastomeric material.
11. The apparatus of embodiment 1, wherein the bracket is secured to the grid plate by a hinged member that is clipped to the grid wall.
12. An apparatus for guiding a biopsy instrument into a breast of a patient supported upon a patient support surface, the apparatus comprising:
   (a) a pair of compressive members movably attached to the patient support surface on opposing sides to locate the patient's breast, a selected one of the pair of compressive members further comprising a grid plate having a plurality of grid apertures defined by a plurality of grid walls, the grid plate being accessible from a lateral side and being at least substantially open from a medial side presented to the breast;
   (b) a bracket comprising a first plate and a second plate, wherein the first plate has a slot defined by two side surfaces and one bottom surface, the slot sheathing a grid wall of the grid plate, and wherein the second plate defines a guide opening; and
   (c) an insert disposed through the guide opening of the bracket, the insert comprising a flange and a shaft, wherein the shaft defines a lumen configured to permit insertion of the biopsy instrument, and wherein the insert is positioned through at least one guide hole of the second plate.
13. The apparatus of embodiment 12, wherein the shaft of the insert is generally cylindrical.
14. The apparatus of embodiment 12, wherein the shaft of the insert is at a non-parallel angle relative to the first plate.
15. The apparatus of embodiment 12, wherein the insert is at least partially constructed of an elastomeric material.
16. The apparatus of embodiment 15, wherein the elastomeric material comprises a material selected from the group consisting of vulcanized thermosetting plastics, thermoplastic elastomers, natural rubber, synthetic rubber, and combinations thereof.
17. A bracket for guiding a biopsy instrument through a grid plate having grid apertures defined by grid walls, the bracket comprising:
   (a) a first plate, the first plate having a slot defined by two side surfaces, wherein the slot is configured to insertingly receive a grid wall of the grid plate; and
   (b) a second plate coupled with the first plate at an angle, the second plate defining a guide opening configured to permit the insertion of the biopsy instrument through the second plate.
18. The bracket of embodiment 17, wherein the angle is a substantially right angle.
19. The bracket of embodiment 17, wherein the first plate and the second plate are formed unitarily together.
20. The bracket of embodiment 17, further comprising an insert member disposed through the guide opening, wherein the insert member comprises a longitudinally extending hollow shaft.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings. In the drawings, like numerals represent like elements throughout the several views.

FIG. 1 is a perspective view of a biopsy system including a control module remotely coupled to a biopsy device, and including a localization assembly.

FIG. 2 is a perspective view of a breast coil of the localization assembly of FIG. 1.

FIG. 3 is a perspective view of the biopsy device inserted through the targeting bracket of the localization assembly of FIG. 1.

FIG. 4 is a perspective view of the obturator and cannula of the biopsy system of FIG. 1.

FIG. 5 is an exploded perspective view of the obturator and cannula of FIG. 4.

FIG. 6 is a perspective view of the obturator and cannula of FIG. 4 with a depth stop device, inserted through the targeting bracket and grid plate of FIG. 1.

FIG. 7 is a perspective view of the targeting bracket of FIG. 1 engaged with the grid plate of the localization assembly of FIG. 1.

FIG. 8 is a perspective view of an exemplary targeting bracket.

FIG. 9 is a side view of the targeting bracket from FIG. 7.

FIG. 10 is a perspective view of an exemplary targeting bracket having three access holes.

FIG. 11 is a perspective view of an exemplary targeting bracket with an insert positioned through an access hole.

FIG. 12 is a side view of an exemplary targeting bracket with an insert having a straight orientation.

FIG. 13 is a side view of the targeting bracket of FIG. 10 with an insert having an angled orientation.

### DETAILED DESCRIPTION

The following description of certain examples should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

Examples described herein relate to a biopsy system, which includes a biopsy instrument positioned and guided through a grid plate by a targeting bracket that is secured to a grid plate wall. As shown in the figures, an exemplary magnetic resonance imaging (MRI or MR imaging) compatible biopsy system may include a control module (12), localization assembly (15), and biopsy device (14). In particular, localization assembly (15) is configured to localize a patient's breast and guide needle (90) of biopsy device (14) to a targeted area within the patient's breast; while control module (12) is operable to control biopsy device (14) after needle (90) has been introduced to the target site. These components and their sub-components will be discussed further below. In addition, targeting brackets for use with various localization assemblies will be discussed. While this disclosure may reference the biopsy system as compatible with MRI and MRI equipment and devices, it should be appreciated that other imaging techniques and equipment and devices may be used with the components described below, including but not limited to stereotactic, ultrasound, PEM, BSGI, and/or other imaging techniques and equipment.

I. Control Module

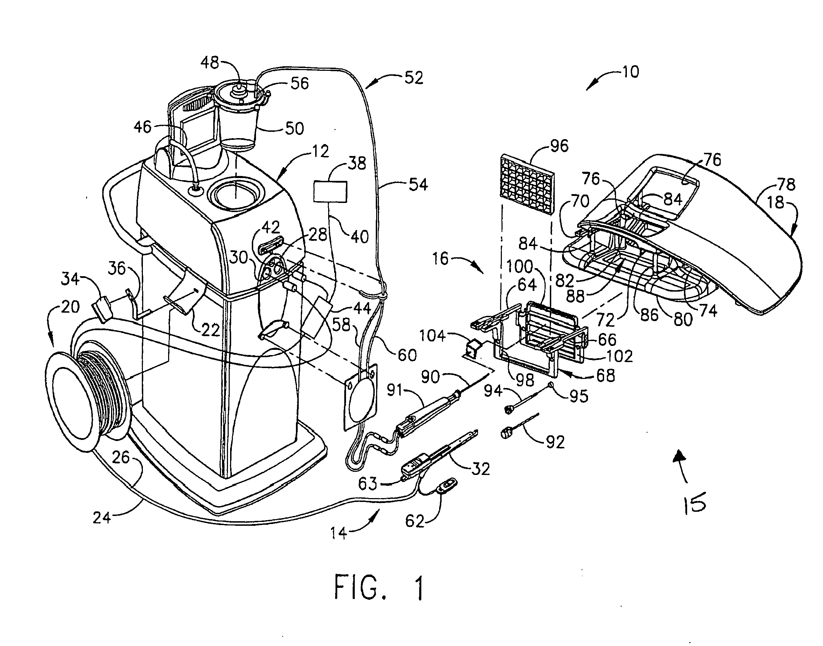

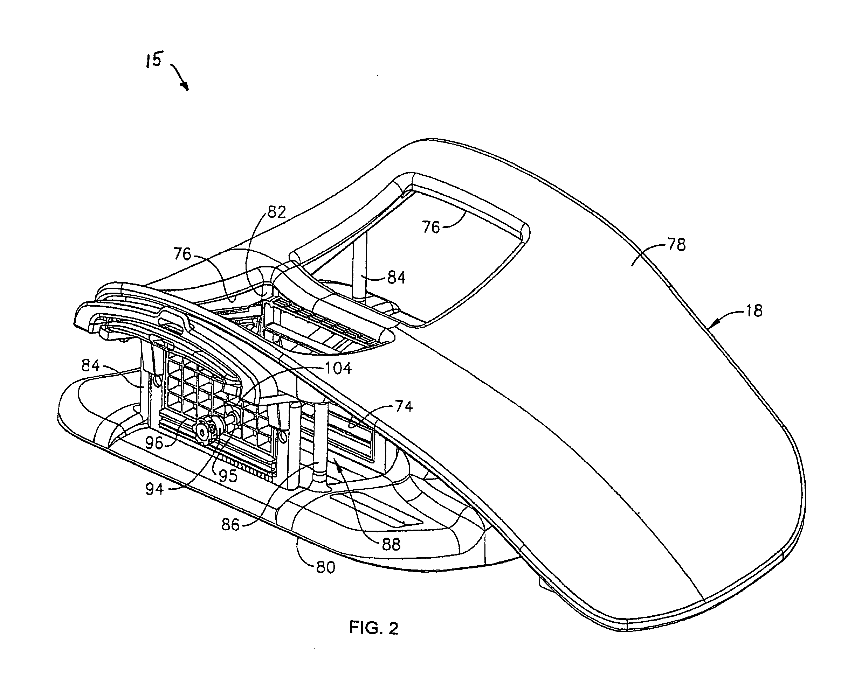

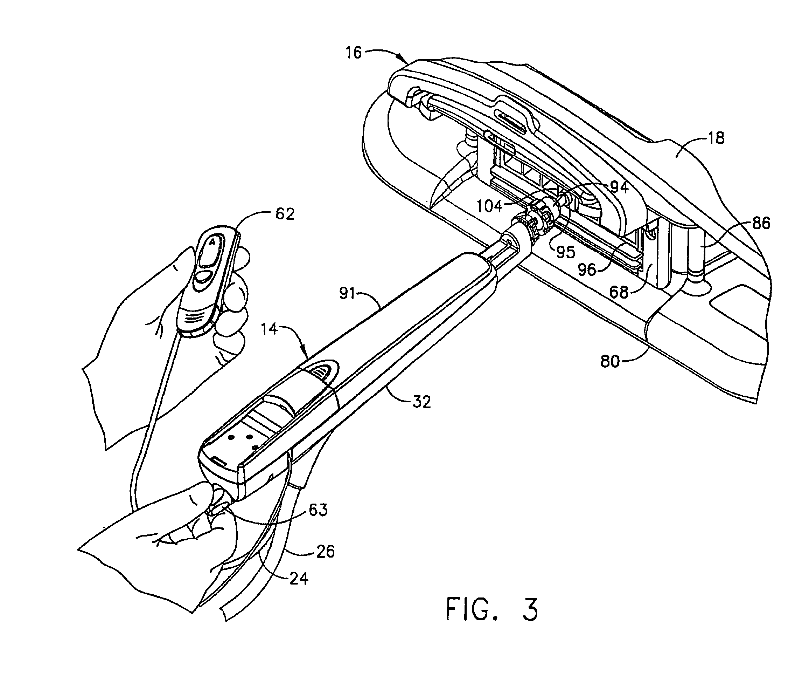

In FIGS. 1-3, MRI compatible biopsy system (10) has control module (12) that may be placed outside of a shielded room containing an MRI machine (not shown) or at least spaced away to mitigate detrimental interaction with its strong magnetic field and/or sensitive radio frequency (RF) signal detection antennas. As described in U.S. Pat. No. 6,752,768, which is hereby incorporated by reference in its entirety, a range of preprogrammed functionality may be incorporated into control module (12) to assist in taking tissue samples. Control module (12) controls and powers biopsy device (14) that is used with localization assembly (15). Biopsy device (14) is positioned and guided by localization fixture (16) attached to breast coil (18) that may be placed upon a gantry (not shown) of a MRI or other imaging machine.

In the present example, control module (12) is mechanically, electrically, and pneumatically coupled to biopsy device (14) so that components may be segregated that need to be spaced away from the strong magnetic field and the sensitive RF receiving components of a MRI machine. Cable management spool (20) is placed upon cable management attachment saddle (22) that projects from a side of control module (12). Wound upon cable management spool (20) is paired electrical cable (24) and mechanical cable (26) for communicating control signals and cutter rotation/advancement motions respectively. In particular, electrical and mechanical cables (24, 26) each have one end connected to respective electrical and mechanical ports (28, 30) in control module (12) and another end connected to holster portion (32) of biopsy device (14). Docking cup (34), which may hold holster portion (32) when not in use, is hooked to control module (12) by docking station mounting bracket (36). It should be understood that such components described above as being associated with control module (12) are merely optional.

Interface lock box (38) mounted to a wall provides tether (40) to lockout port (42) on control module (12). Tether (40) is uniquely terminated and of short length to preclude inadvertent positioning of control module (12) too close to a MRI machine or other machine. In-line enclosure (44) may register tether (40), electrical cable (24) and mechanical cable (26) to their respective ports (42, 28, 30) on control module (12).

Vacuum assist is provided by first vacuum line (46) that connects between control module (12) and outlet port (48) of vacuum canister (50) that catches liquid and solid debris. Tubing kit (52) completes the pneumatic communication between control module (12) and biopsy device (14). In particular, second vacuum line (54) is connected to inlet port (56) of vacuum canister (50). Second vacuum line (54) divides into two vacuum lines (58, 60) that are attached to biopsy device (14). With biopsy device (14) installed in holster portion (32), control module (12) performs a functional check. Saline may be manually injected into biopsy device (14) or otherwise introduced to biopsy device (14), such as to serve as a lubricant and to assist in achieving a vacuum seal and/or for other purposes. Control module (12) actuates a cutter mechanism (not shown) in biopsy device (14), monitoring full travel of a cutter in biopsy device (14) in the present example. Binding in mechanical cable (26) or within biopsy device (14) may optionally monitored with reference to motor force exerted to turn mechanical cable (26) and/or an amount of twist in mechanical cable (26) sensed in comparing rotary speed or position at each end of mechanical cable (26).

Remote keypad (62), which is detachable from holster portion (32), communicates via electrical cable (24) to control panel (12) to enhance clinician control of biopsy device (14) in the present example, especially when controls that would otherwise be on biopsy device (14) itself are not readily accessible after insertion into localization fixture (16) and/or placement of control module (12) is inconveniently remote (e.g., 30 feet away). However, as with other components described herein, remote keypad (62) is merely optional, and may be modified, substituted, supplemented, or omitted as desired. In the present example, aft end thumbwheel (63) on holster portion (32) is also readily accessible after insertion to rotate the side from which a tissue sample is to be taken.

Of course, the above-described control module (12) is merely one example. Any other suitable type of control module (12) and associated components may be used. By way of example only, control module (12) may instead be configured and operable in accordance with the teachings of U.S. Pub. No. 2008/0228103, entitled "Vacuum Timing Algorithm for Biopsy Device," published September 18, 2008, the disclosure of which is incorporated by reference herein. As another merely illustrative example, control module (12) may instead be configured and operable in accordance with the teachings of U.S. Patent Application Serial No. 12/337,814, entitled "Control Module Interface for MRI Biopsy Device," filed December 18, 2008, the disclosure of which is incorporated by reference herein. Alternatively, control module (12) may have any other suitable components, features, configurations, functionalities, operability, etc. Other suitable variations of control module (12) and associated components will be apparent to those of ordinary skill in the art in view of the teachings herein.

II. Localization Assembly

Localization assembly (15) of the present example comprises breast coil (18) and localization fixture (16). These components of localization assembly (15) are described further below.

Left and right parallel upper guides (64, 66) of localization framework (68) are laterally adjustably received respectively within left and right parallel upper tracks (70, 72) attached to under side (74) and to each side of a selected breast aperture (76) formed in patient support platform (78) of breast coil (18). Base (80) of breast coil (18) is connected by centerline pillars (82) that are attached to patient support platform (78) between breast apertures (76). Also, a pair of outer vertical support pillars (84, 86) on each side spaced about a respective breast aperture (76) respectively define lateral recess (88) within which localization fixture (16) resides.

It should be appreciated that the patient's breasts hang pendulously respectively into breast apertures (76) within lateral recesses (88) in the present example. For convenience, herein a convention is used for locating a suspicious lesion by Cartesian coordinates within breast tissue referenced to localization fixture (16) and to thereafter selectively position an instrument, such as needle (90) of probe (91) that is engaged to holster portion (32) to form biopsy device (14). Of course, any other type of coordinate system or targeting techniques may be used. To enhance hands-off use of biopsy system (10), especially for repeated re-imaging within the narrow confines of a closed bore MRI machine, biopsy system (10) may also guide obturator (92) encompassed by cannula (94). Depth of insertion is controlled by depth stop device (95) longitudinally positioned on either needle (90) or cannula (94). Alternatively, depth of insertion may be controlled in any other suitable fashion.

This guidance is specifically provided by a lateral fence in the present example, depicted as grid plate (96), which is received within laterally adjustable outer three-sided plate bracket (98) attached below left and right parallel upper guides (64, 66). Similarly, a medial fence with respect to a medial plane of the chest of the patient, depicted as medial plate (100), is received within inner three-sided plate bracket (102) attached below left and right parallel upper guides (64, 66) close to centerline pillars (82) when installed in breast coil (18). To further refine the insertion point of the instrument (e.g., needle (90) of probe (91), obturator/cannula (92, 94), etc.), targeting bracket (104) may be inserted into grid plate (96).

In the present example, the selected breast is compressed along an inner (medial) side by medial plate (100) and on an outer (lateral) side of the breast by grid plate (96), the latter defining an X-Y plane. The X-axis is vertical (sagittal) with respect to a standing patient and corresponds to a left-to-right axis as viewed by a clinician facing the externally exposed portion of localization fixture (16). Perpendicular to this X-Y plane extending toward the medial side of the breast is the Z-axis, which typically corresponds to the orientation and depth of insertion of needle (90) or obturator/cannula (92, 94) of biopsy device (14). For clarity, the term Z-axis may be used interchangeably with "axis of penetration", although the latter may or may not be orthogonal to the spatial coordinates used to locate an insertion point on the patient. Versions of localization fixture (16) described herein allow a non-orthogonal axis of penetration to the X-Y axis to a lesion at a convenient or clinically beneficial angle.

It should be understood that the above-described localization assembly (15) is merely one example. Any other suitable type of localization assembly (15) may be used, including but not limited to localization assemblies (15) that use a breast coil (18) and/or localization fixture (16) different from those described above. Other suitable components, features, configurations, functionalities, operability, etc. for a localization assembly (15) will be apparent to those of ordinary skill in the art in view of the teachings herein.

III. Biopsy Device

As shown in FIG. 1, one version of biopsy device (14) may comprise holster portion (32) and probe portion (91). Exemplary holster portion (32) was discussed previously in the above section addressing control module (12). The following paragraphs will discuss probe (91) and associated components and devices in further detail.

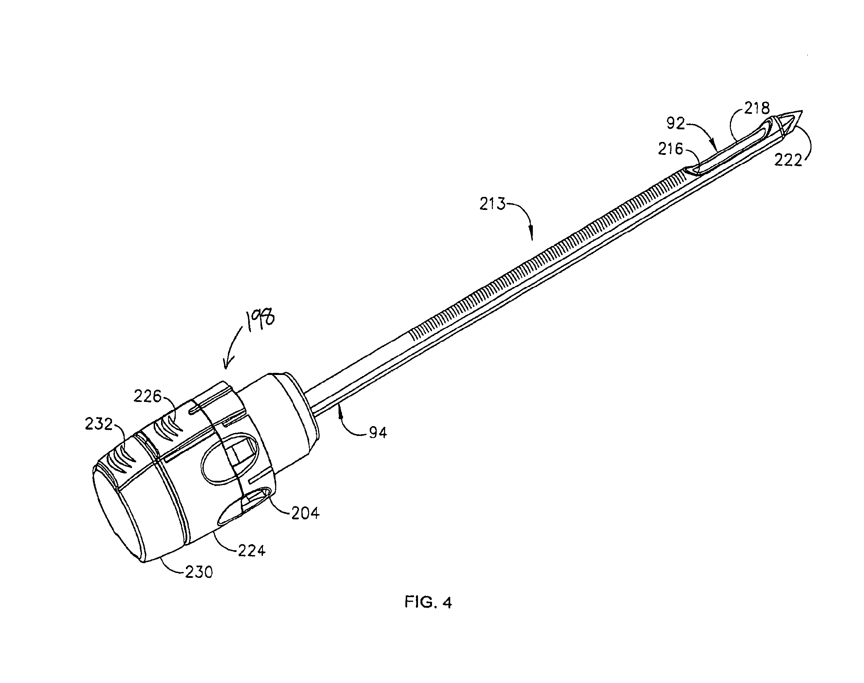

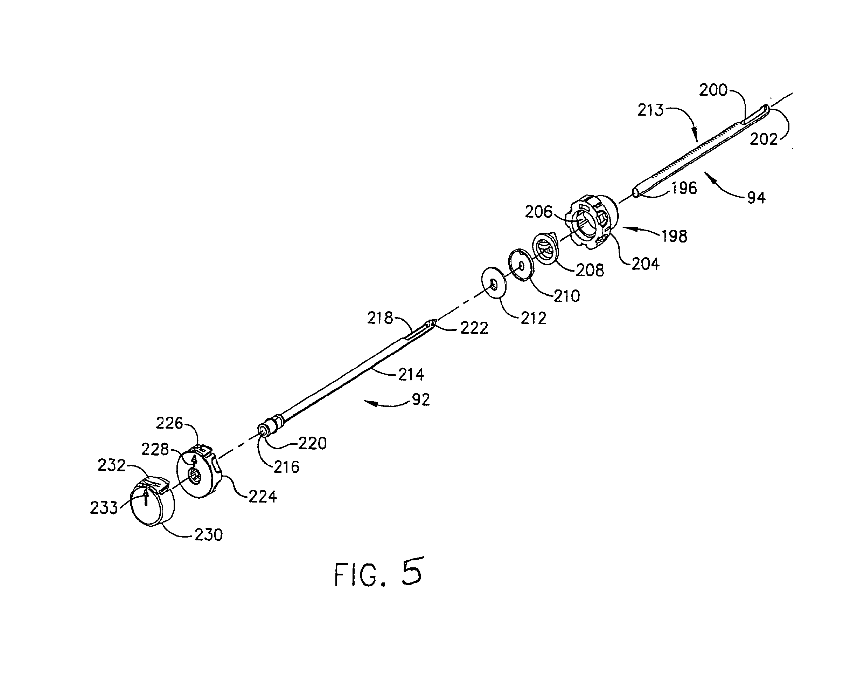

In the present example, cannula (94) and obturator (92) are associated with probe (91). In particular, and as shown in FIGS. 4, 5, and 6, obturator (92) is slid into cannula (94) and the combination is guided through targeting bracket (104) to the biopsy site within the breast tissue. Obturator (92) is then withdrawn from cannula (94), then needle (90) of probe (91) is inserted in cannula (94), and then biopsy device (14) is operated to acquire one or more tissue samples from the breast via needle (90).

Cannula (94) of the present example is proximally attached to cylindrical hub (198) and cannula (94) includes lumen (196) and lateral aperture (200) proximate to open distal end (202). Cylindrical hub (198) has exteriorly presented thumbwheel (204) for rotating lateral aperture (200). Cylindrical hub (198) has interior recess (206) that encompasses duckbill seal (208), wiper seal (210) and seal retainer (212) to provide a fluid seal when lumen (196) is empty and for sealing to inserted obturator (92). Longitudinally spaced measurement indicia (213) along an outer surface of cannula (94) visually, and perhaps physically, provide a means to locate depth stop device (95) of FIG. 1.

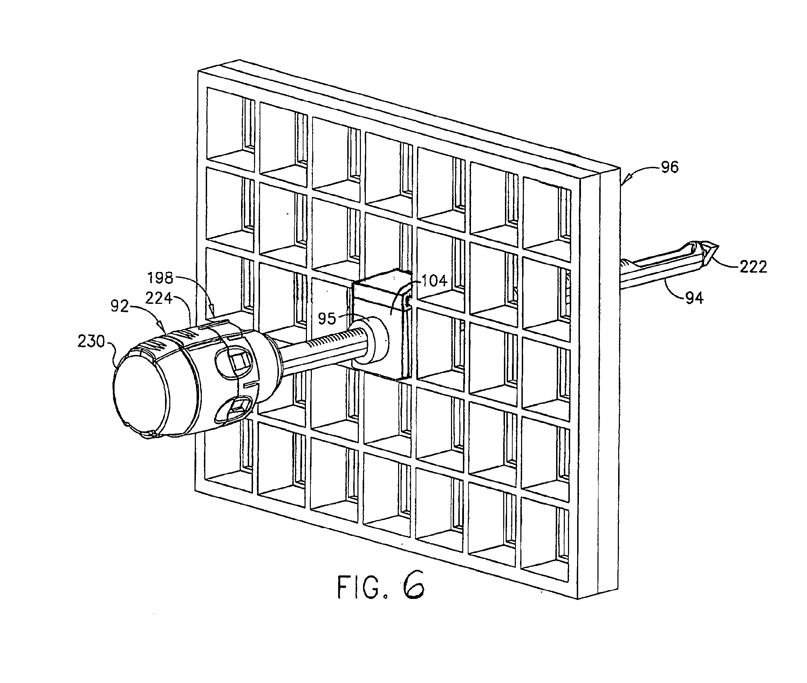

Obturator (92) of the present example incorporates a number of components with corresponding features. Hollow shaft (214) includes fluid lumen (216) that communicates between imageable side notch (218) and proximal port (220). Hollow shaft (214) is longitudinally sized to extend, when fully engaged with cannula (94), piercing tip (222) out of distal end (202) of cannula (94). Obturator thumbwheel cap (224) encompasses proximal port (220) and includes locking feature (226), which includes visible angle indicator (228), that engages cannula thumbwheel (204) to ensure that imageable side notch (218) is registered to lateral aperture (200) in cannula (94). Obturator seal cap (230) may be engaged proximally into obturator thumbwheel cap (224) to close fluid lumen (216). Obturator seal cap (230) of the present example includes locking or locating feature (232) that includes visible angle indicator (233) that corresponds with visible angle indicator (228) on obturator thumbwheel cap (224), which may be fashioned from either a rigid, soft, or elastomeric material. In FIG. 6, targeting bracket (104) has guided obturator (92) and cannula (94) through grid plate (96).

While obturator (92) of the present example is hollow, it should be understood that obturator (92) may alternatively have a substantially solid interior, such that obturator (92) does not define an interior lumen. In addition, obturator (92) may lack side notch (218) in some versions. Other suitable components, features, configurations, functionalities, operability, etc. for an obturator (92) will be apparent to those of ordinary skill in the art in view of the teachings herein. Likewise, cannula (94) may be varied in a number of ways. For instance, in some other versions, cannula (94) has a closed distal end (202). As another merely illustrative example, cannula (94) may have a closed piercing tip (222) instead of obturator (92) having piercing tip (222). In some such versions, obturator (92) may simply have a blunt distal end; or the distal end of obturator (92) may have any other suitable structures, features, or configurations. Other suitable components, features, configurations, functionalities, operability, etc. for a cannula (94) will be apparent to those of ordinary skill in the art in view of the teachings herein. Furthermore, in some versions, one or both of obturator (92) or cannula (94) may be omitted altogether. For instance, needle (90) of probe (91) may be directly inserted into a guide cube (104), without being inserted into guide cube (104) via cannula (94).

Another component that may be used with cannula (94) and/or needle (90) is depth stop (95). Depth stop may be of any suitable configuration that is operable to prevent cannula (94) and obturator (92) (or needle (90)) from being inserted further than desired. For instance, depth stop (95) may be positioned on the exterior of cannula (94) (or needle (94)), and may be configured to restrict the extent to which cannula (94) is inserted into a targeting bracket. It should be understood that such restriction by depth stop (95) may further provide a limit on the depth to which the combination of cannula (94) and obturator (92) (or needle (90)) may be inserted into the patient's breast. Furthermore, it should be understood that such restriction may establish the depth within the patient's breast at which biopsy device (14) acquires one or more tissue samples after obturator (92) has been withdrawn from cannula (94) and needle (90) has been inserted in cannula (94). Exemplary depth stops (95) that may be used with biopsy system (10) are described in U.S. Pub. No. 2007/0255168, entitled "Grid and Rotatable Cube Guide Localization Fixture for Biopsy Device," published November 1, 2007, the disclosure of which is incorporated by reference herein; and U.S. Pat. No. http://patft1.uspto.gov/netacgi/nph-Parser?Sect1=PTO1&Sect2=HITOFF&d=PALL&p=1&u=%2Fnetahtml%2FPTO %2Fsrchnum.htm&r=1&f=G&1=50&s1=7507210.PN.&OS=PN/7507210&RS=P N/7507210 - h0#h0http://patnl.uspto.gov/netacgi/nph-Parser?Sect1=PTO1&Sect2=HITOFF&d=PALL&p=1&u=%2Fnetahtml%2FPTO %2Fsrchnum.htm&r=1&f=G&1=50&s1=7507210.PN.&OS=PN/7507210&RS=P N/7507210 - h2#h27,507,210, entitled "Biopsy Cannula Adjustable Depth Stop," issued March 24, 2009, the disclosure of which is incorporated by reference herein.

In the present example, and as noted above, biopsy device (14) includes a needle (90) that may be inserted into cannula (94) after the combination of cannula (94) and obturator (92) has been inserted to a desired location within a patient's breast and after obturator (92) has been removed from cannula (94). Needle (90) of the present example comprises a lateral aperture (not shown) that is configured to substantially align with lateral aperture (200) of cannula (94) when needle (90) is inserted into lumen (196) of cannula (94). Probe (91) of the present example further comprises a rotating and translating cutter (not shown), which is driven by components in holster (32), and which is operable to sever tissue protruding through lateral aperture (200) of cannula (94) and the lateral aperture of needle (90). Severed tissue samples may be retrieved from biopsy device (14) in any suitable fashion.

By way of example only, biopsy device (14) may be configured and operable in accordance with the teachings of U.S. Pub. No. 2008/0228103, entitled "Vacuum Timing Algorithm for Biopsy Device," published September 18, 2008, the disclosure of which is incorporated by reference herein. As another merely illustrative example, biopsy device (14) may be configured and operable in accordance with the teachings of U.S. Patent Application Serial No. 12/337,874, entitled "Mechanical Tissue Sample Holder Indexing Device," filed December 18, 2008, the disclosure of which is incorporated by reference herein. As another merely illustrative example, biopsy device (14) may be configured and operable in accordance with the teachings of U.S. Patent Application Serial No. 12/337,674, entitled "Biopsy Device with Sliding Cutter Cover," filed December 18, 2008, the disclosure of which is incorporated by reference herein. By way of example only, cannula (94) may be replaced with any of the detachable needles described in U.S. Patent Application Serial No. 12/337,674, entitled "Biopsy Device with Sliding Cutter Cover." As another merely illustrative example, biopsy device (14) may be configured and operable in accordance with the teachings of U.S. Patent Application Serial No. 12/337,911, entitled "Biopsy Device with Discrete Tissue Chambers," filed December 18, 2008, the disclosure of which is incorporated by reference herein. As another merely illustrative example, biopsy device (14) may be configured and operable in accordance with the teachings of U.S. Patent Application Serial No. 12/337,942, entitled "Biopsy Device with Central Thumbwheel," filed December 18, 2008, the disclosure of which is incorporated by reference herein. Alternatively, biopsy device (14) may have any other suitable components, features, configurations, functionalities, operability, etc. Other suitable variations of biopsy device (14) and associated components will be apparent to those of ordinary skill in the art in view of the teachings herein.

IV. Targeting Bracket

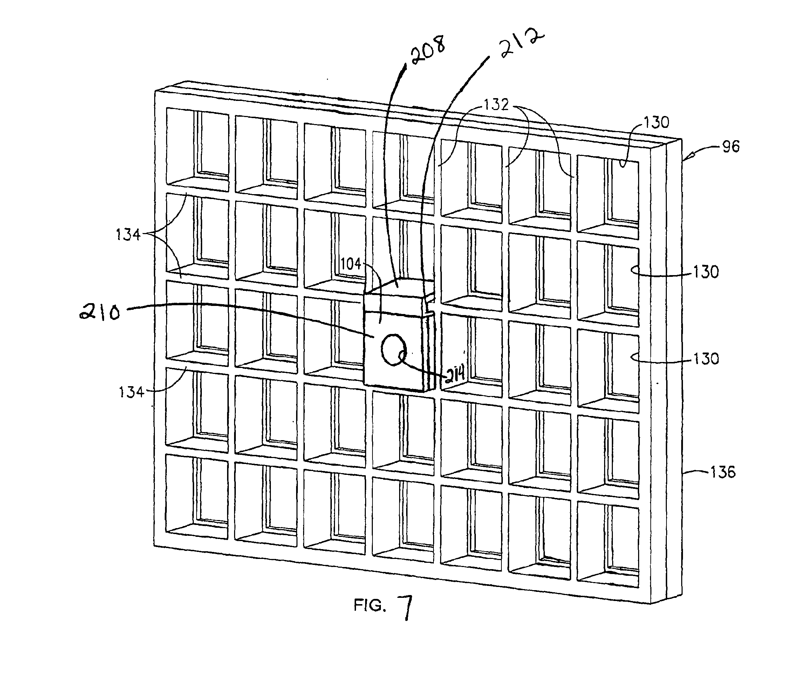

The targeting brackets described below and shown in FIGS. 7-13 are generally configured for use with a localization assembly (15) as described above. In particular, examples of targeting brackets are configured to engage vertical grid walls (132) and/or horizontal grid walls (134) of grid plate (96), such as to provide insertion guidance for a combined obturator (92) and cannula (94) and/or for a needle (90). Numerous features of merely exemplary targeting brackets will be discussed in the paragraphs that follow, while other suitable features and examples of targeting brackets will be apparent to those of ordinary skill in the art in view of the teachings herein.

In some versions, targeting brackets may comprise a body defined by one or more plates. The body may include one or more guide holes or other types of passages that are formed through plates of the targeting bracket and that may be used to guide an instrument such as a biopsy device (14) or a portion of a biopsy device (14) (e.g., needle (90) of biopsy device (14), a combination of cannula (94) and obturator (92), etc.). Targeting brackets may be rotatable about one, two, or three axes to position the one or more guide holes or passages of the targeting bracket into a desired position.

Referring now to FIG. 7, one example of a targeting bracket (104) is shown engaged with a grid plate (96). Grid plate (96) of this example comprises intersecting grid plate walls, such as vertical grid walls (132) and horizontal grid walls (134), which define grid apertures (130) through which a biopsy device (14) or a portion of a biopsy device (14) (e.g., needle (90) of biopsy device (14), a combination of cannula (94) and obturator (92), etc.) may be guided. The targeting bracket (104) may be secured to a vertical grid wall (132) or a horizontal grid wall (134). By way of example only, in the illustrative version shown in FIGS. 6-7, targeting bracket (104) is shown engaged with a horizontal grid wall (134). It should be understood, however, that targeting bracket (104) may be coupled with a variety of other types of localization fixtures, including but not limited to those having only vertical walls or only horizontal walls, among other types.

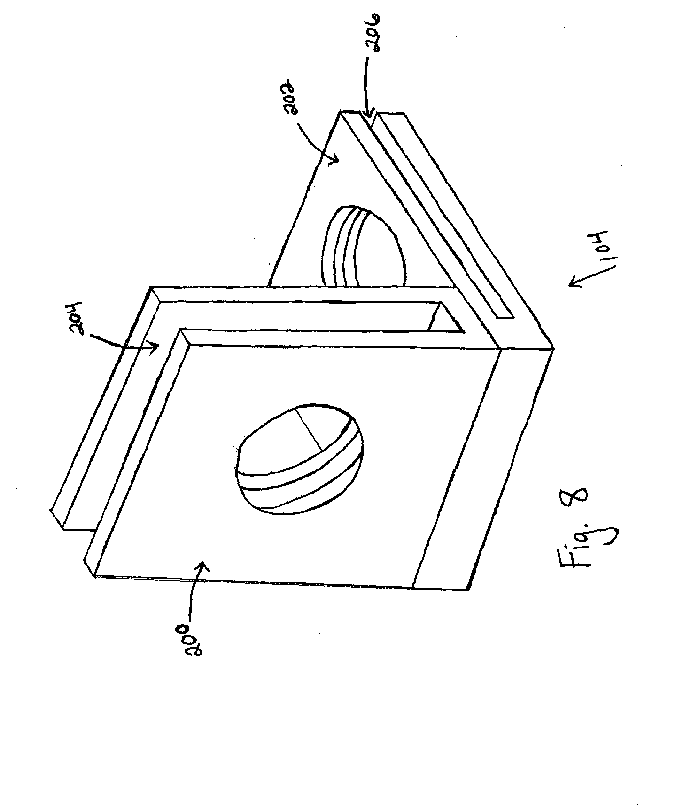

FIGS. 8-10 show the targeting bracket (104) of the present example in more detail. Targeting bracket (104) of this example comprises a plurality of plates. In the illustrated version, FIG. 8 shows targeting bracket (104) as including a first plate (200) and a second plate (202). First plate (200) and second plate (202) may be positioned at a substantially right angle relative to each other such that they form an L-shape. Of course, first plate (200) and second plate (202) may instead be positioned at a substantially non-perpendicular angle relative to each other, such as to permit angled targeting of the biopsy tissue. First plate (200) and second plate (202) may be secured together, for example, by a nail, screw, bolt, adhesive, glue, weld, snap-fitting, or using any other suitable devices, structures, or techniques. Alternatively, plates (200, 202) may be integrally and simultaneously formed together as a single unitary structure. In some other versions, targeting bracket (104) may comprise more than two plates. For example, a third plate (not pictured) may be positioned opposite of but otherwise parallel to plate (200) (e.g., such that targeting bracket (104) forms a "U" shape. As another merely illustrative example, a third plate may be positioned adjacent to each plate (200, 202), such that the three plates all meet at a common corner. Alternatively, targeting bracket (104) may have any other suitable number of plates in any suitable arrangement, and may have any other suitable components, features, configurations, functionalities, operability, etc. Other suitable variations of targeting bracket (104) and associated components will be apparent to those of ordinary skill in the art in view of the teachings herein.

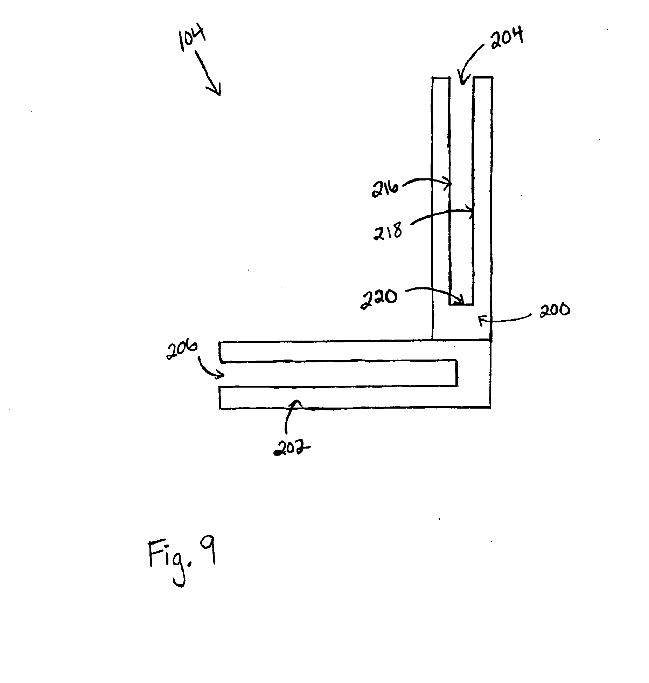

In some versions, at least one plate includes a slot or cutout into which a grid plate wall may be positioned. For example, FIGS. 8-9 show two plates (200) and (202), each comprising a respective slot - plate (200) comprising a first slot (204) and plate (202) comprising a second slot (206). Alternatively, FIG. 6 shows a targeting bracket (104) comprising a first plate (208) and second plate (210), wherein only first plate (208) includes a slot (212). As will be appreciated by one of ordinary skill in the art in view of the teachings herein, a slot (e.g., (204, 206), or (212)) may have any suitable cross-sectional shape that accepts a vertical grid wall (132) or horizontal grid wall (134) such that the grid wall (132, 134) is at least partially sheathed by the plate. For example, slot (204) shown in FIG. 9 has a generally U-shaped cross-section created by a bottom surface (220) and diametrically opposed side surfaces (216, 218) that are substantially perpendicular to the bottom surface (220). While surfaces (216, 218) both extend to a common length in the present example, it should be understood that one surface (e.g., surface (218)) may be longer than the other surface (e.g., surface (216)), or that surfaces (216, 218) may have any other suitable dimensions or relationships. Targeting bracket (104) may be secured to grid plate (96) by the friction between the surfaces (216, 218) of a slot (204, 206) and the surface of a grid wall (132, 134). Accordingly, in some versions, the dimensions of a slot (204, 206) may be determined at least in part based on the thickness of the vertical grid wall (132) or horizontal grid wall (134) that the slot (204, 206) sheaths, among other factors.

In the present example, surfaces (216, 218) are substantially smooth and parallel to each other. In some versions, surfaces (216, 218) are slightly inclined inwardly toward each other, such that the outer ends of surfaces (216, 218) are closer to each other than the ends of surfaces (216, 218) that are adjacent to surface (220). In particular, the outer ends of surfaces (216, 218) may define a gap that is narrower than the thickness of grid walls (132, 134). Targeting bracket (104) may be formed of a resilient material, such that surfaces (216, 218) are biased toward this inclined configuration yet such that surfaces (216, 218) may be spread further apart. Such a configuration may provide an interference fit with a grid wall (132, 134) such as to improve gripping of grid wall (132, 134) by targeting bracket (104). In some such versions, or other versions described herein, the corners at the outer ends of surfaces (216, 218) may be chamfered or rounded, etc., to facilitate insertion of grid wall (132, 134) into slot (204). In still other versions (including but not limited to those where surfaces (216, 218) are substantially parallel), surfaces (216, 218) include a material having elastomeric properties or other properties configured to facilitate gripping of grid wall (132, 134) by bracket (104). In addition or in the alternative, surfaces (216, 218) may include ridges, knurling, bump-like protrusions, and/or other features configured to facilitate gripping of grid wall (132, 134) by bracket (104). Still other suitable features or configurations of surfaces (216, 218) will be apparent to those of ordinary skill in the art in view of the teachings herein.

Alternatively, a targeting bracket (104) may be removably secured to a grid plate (96) in any other suitable fashion as will be apparent to one of ordinary skill in the art in view of the teachings herein. For example, a targeting bracket (104) may be secured to grid plate (96) by a living hinge or other feature that is clipped onto or otherwise engaged with a wall (132, 134) of the grid plate (96). The at least one slot included in targeting bracket (104) of the present example permits the bracket (104) to be positioned onto grid plate (96) such that the at least one slot sheaths or otherwise engages a grid wall (132, 134) and removably secures the targeting bracket (104) in place. In particular, such engagement is secure enough to permit a biopsy device (14) to be supported by targeting bracket (104) when engaged with grid plate (96); while also permitting targeting bracket (104) to be removed from grid plate (96) and repositioned (e.g., to a different rotational orientation and/or to be engaged with a different grid wall (132, 134), etc.).

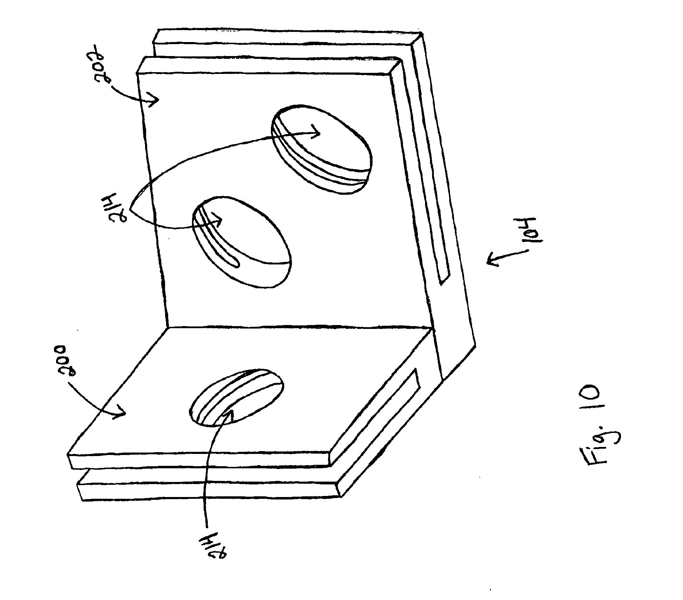

In the present example, at least one plate of targeting bracket (104) comprises a guide hole or other feature through which an instrument, such as an obturator (92) and cannula (94) or needle (90), of biopsy device (14) may be inserted. As will be apparent to one of ordinary skill in the art in view of the teachings herein, a targeting bracket (104) may comprise any suitable number of guide holes or passages in any suitable arrangement to permit access to various portions of tissue. For example, FIG. 10 shows a version of targeting bracket (104) wherein a first plate (200) includes one guide hole (214), and a second plate (202) includes two guide holes (214). Alternatively, targeting bracket (104) may comprise only one guide hole (214). For example, FIG. 7 shows a targeting bracket (104) comprising only one guide hole (214) located within plate (210). Alternatively, any other suitable number of guide holes (214) may be provided by a targeting bracket (104) in any suitable arrangement.

Due to the substantially L-shaped configuration of first plate (200) and second plate (202) in the illustrated version, when the slot (204, 206) of one plate (200, 202) sheaths a grid wall (132, 134), the surface of the other plate (202, 200) extends in front of grid plate (96) such that the plate (202, 200) at least partially covers a grid aperture (130). For example, as shown in FIG. 7, the sheathing of horizontal grid wall (134) by slot (212) causes plate (210) to cover a grid aperture (130). In some other versions, such as but not limited to those where first plate (200) and second plate (202) are positioned at a substantially non-perpendicular angle relative to each other, the engagement of a targeting bracket (104) with a grid plate (96) may cause only a partial covering of grid aperture (130). Furthermore, while the shape of the guide holes (214) of the present example are substantially circular, it should be understood that guide holes (214) may take any other suitable shape that permits the insertion of an instrument, such as an obturator (92) and cannula (94) or needle (90), etc.

It will also be appreciated based on the teachings herein that while orthogonal guide holes (214) may be used in some versions, angled guide holes (not pictured) may be used instead of or in addition to orthogonal guide holes in other versions. A guide hole (214) may permit the orthogonal and/or non-orthogonal positioning of an instrument, such as an obturator (92) and cannula (94) or needle (90), through a targeting bracket (104), such that a greater area of tissue and/or angular orientations is/are available for insertion and/or removal than if the instrument was limited to a perpendicular insertion. Thus, while FIG. 6 shows an orthogonal insertion of an obturator (92), it should be understood that one or more guide holes (214) may provide for an angle of insertion that is non-perpendicular relative to its corresponding plate (200, 202) (e.g., such that the angle of insertion is non-perpendicular relative to the plane defined by grid plate (96), etc.). For instance, the interior portion of plate (200, 202) that defines guide hole (214) may be angled, such that guide hole (214) has an angled perimeter, with such an angled configuration providing an angled guide for a portion of biopsy device (14) (e.g., needle (90) of biopsy device (14), a combination of cannula (94) and obturator (92), etc.) that is inserted through guide hole (214). In addition or in the alternative, the parts of guide hole (214) formed through opposing side surfaces (216, 218) of targeting bracket (104) may be offset relative to each other, such that the two parts of guide hole (214) are not coaxially aligned. Again, the edges of such offset guide hole (214) parts may be angled, rounded, chamfered, or otherwise configured to provide for relatively smooth insertion of a portion of biopsy device (14) (e.g., needle (90) of biopsy device (14), a combination of cannula (94) and obturator (92), etc.) through both offset parts of guide hole (214). In addition or in the alternative, and as noted above, plates (200, 202) may be non-perpendicular relative to each other to provide an angle of insertion that is non-perpendicular relative to the plane defined by grid plate (96). Still other ways in which guide holes (214) and/or other aspects of a targeting bracket (104) may be configured to provide an angle of insertion that is non-perpendicular relative to the plane defined by grid plate (96) will be apparent to those of ordinary skill in the art in view of the teachings herein.

In addition, it should be understood that the location of a guide hole (104) within a plate (200, 202) may affect the area of tissue accessible by an instrument. Based on the teachings herein, it will be appreciated that one or more guide holes (214) may be positioned in any suitable location(s) within a plate (200, 202), such as at the center, at one or more corners, at one or more positions that is/are offset from the center and/or corners, etc. In the illustrated version as shown in FIG. 10, guide hole (214) of plate (200) is in a generally centered location in plate (200), while guide holes (214) of plate (202) are positioned closer to the corners of plate (202). As another merely illustrative example, two or more guide holes (214) in a plate (202) may partially overlap each other, such as the overlapping guide holes shown in FIG. 18 of U.S. Pat. No. http://patftl.uspto.gov/netacgi/nph-Parser?Sect1=PTO1&Sect2=HITOFF&d=PALL&p= 1&u=%2Fnetahtml%2FPTO %2Fsrchnum.htm&r=1&f=G&1=50&s1=7507210.PN.&OS=PN/7507210&RS=P N/7507210 - h0#h0http://patftl.uspto.gov/netacgi/nph-Parser?Sect1=PTO1&Sect2=HITOFF&d=PALL&p=1&u=%2Fnetahtml%2FPTO %2Fsrchnum.htm&r=1&f=G&1=50&s1=7507210.PN.&OS=PN/7507210&RS=P N/7507210 - h2#h27,507,210, entitled "Biopsy Cannula Adjustable Depth Stop," issued March 24, 2009, the disclosure of which is incorporated by reference herein. Other suitable positions and arrangements of guide holes (214) will be apparent to those of ordinary skill in the art in view of the teachings herein.

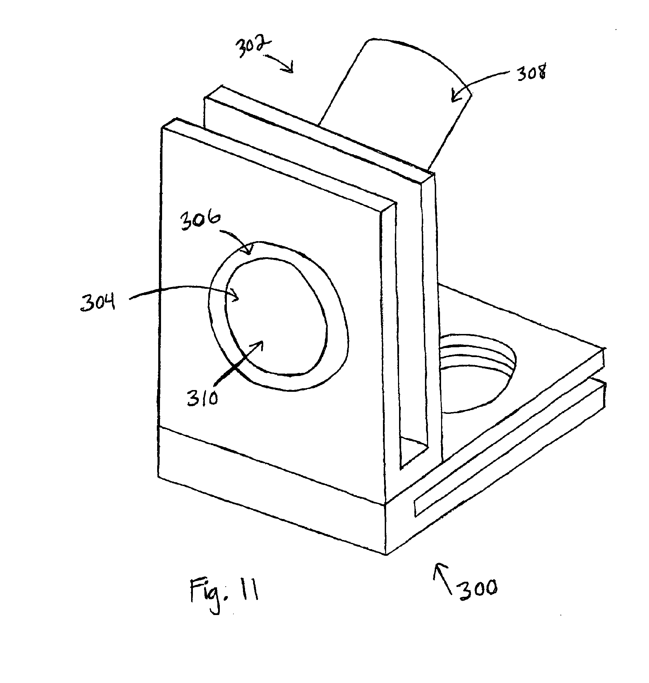

Localization assembly (15) may further comprise an insert positioned within a guide hole of the targeting bracket. For example, in FIG. 11, an insert (302) is shown positioned through guide hole (304) of a targeting bracket (300). Such an insert (302) may be added to a biopsy system (10) to provide further stabilization of an instrument, such as an obturator (92) and cannula (94) or needle (90) that is inserted in targeting bracket (300). For example, with some versions of a targeting bracket (104) that merely comprise guide holes (214) that do not extend the full depth of the grid plate (96), movement of an instrument positioned through a guide hole (214) (e.g., angular deflection, longitudinal slipping, etc.) may be relatively easy. An insert (302) may reduce or prevent such additional movement by extending at the full depth of a grid plate (96) (or less than or greater than the full depth of grid plate (96)). Insert (302) of the present example comprises a flange (306) and a shaft (308). Flange (306) forms a lip to surround guide hole (304) and thereby prevents insert (302) from pushing through the guide hole (304). Shaft (308) extends from flange (306) and through the guide hole (304). In the illustrated version, shaft (308) includes a lumen (310) through which an instrument, such as an obturator (92) and cannula (94) or needle (90), may be positioned. While the shaft (308) of the present example is generally cylindrical, it should be understood that shaft (308) may alternatively take any suitable shape as will be apparent to one of ordinary skill.

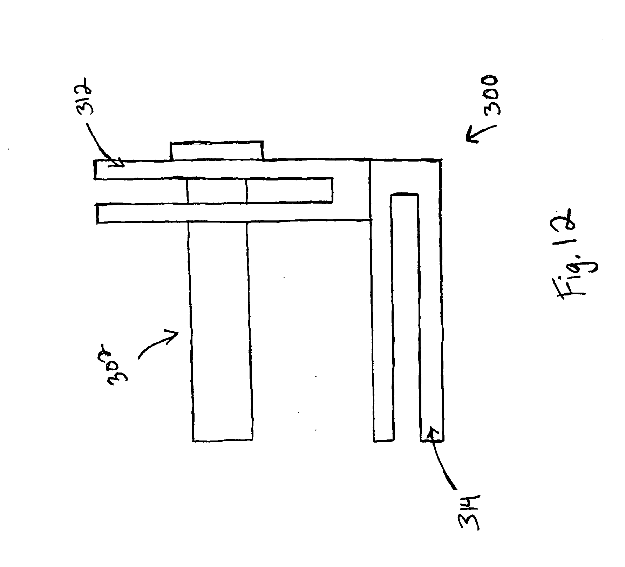

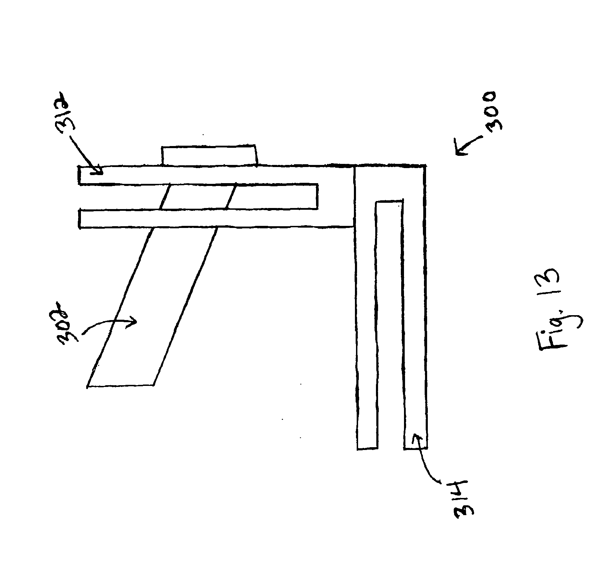

An insert (302) can have a straight or angled orientation. For example, FIG. 12 shows an insert (302) positioned through a guide hole in a first plate (312) in a substantially straight orientation such that it is generally parallel to a second plate (314). A straight orientation of insert (302) allows for the orthogonal positioning of an instrument through the insert (302). Alternatively, the insert (302) shown in FIG. 13 has a substantially angled orientation, such that it is generally at a non-parallel angle relative to a second plate (314). An angled orientation allows for the non-orthogonal positioning of an instrument through the insert (302). In addition, an angled insert (302) can be rotated within plate (312) to provide different angular orientations, such as to provide access to different areas of the tissue.

Insert (302) may be made of any suitable material as will be apparent to one ordinary skill in the art. For example, insert (302) may comprise an elastomeric polymer or rubber material. By way of example only, suitable elastomeric materials may include thermosetting plastics that may require vulcanization, thermoplastic elastomers (e.g., Santoprene^{™} among others), natural rubber, synthetic rubbers (e.g. ethylene propylene diene M-class-EPDM-among others), and other polymers having suitable elastic properties. In some versions, the entire body of insert (302) may be elastomeric. In other versions, insert (302) may be only partially elastomeric, by, for example, having an elastomeric interior. As another merely illustrative example, flange (306) may be elastomeric while shaft (308) is non-elastomeric, or vice-versa. Portions of insert (302) having elastomeric properties or similar properties may reduce the likelihood that in instrument (e.g., needle (90) of biopsy device (14), a combination of cannula (94) and obturator (92), etc.) that is inserted through insert (302) will inadvertently slip along its longitudinal axis, inadvertently rotate about its longitudinal axis, etc. It should be understood that the above-described insert (302) is merely one example. Insert (302) may have any other suitable components, features, configurations, functionalities, operability, etc. Other suitable variations of insert (302) and associated components will be apparent to those of ordinary skill in the art in view of the teachings herein. Of course, as with other components of the localization assembly (15) described herein, insert (302) is merely optional, and may be modified, substituted, supplemented, or omitted as desired.

It should also be understood that, even in versions of targeting bracket (104) that lack insert (302), at least a portion of each guide hole (214) may be lined with a material having elastomeric properties or other properties configured to facilitate gripping of by bracket (104) of an instrument (e.g., needle (90) of biopsy device (14), a combination of cannula (94) and obturator (92), etc.) that is inserted through guide hole (214). Such a modification to bracket (104) may reduce the likelihood that the instrument inserted through guide hole (214) will inadvertently slip along its longitudinal axis, inadvertently rotate about its longitudinal axis, etc. In addition or in the alternative, targeting bracket (104) may be provided with any other suitable components, features, configurations, functionalities, operability, etc., as will be apparent to those of ordinary skill in the art in view of the teachings herein.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the present invention have application in conventional endoscopic and open surgical instrumentation as well as application in robotic-assisted surgery.

Versions of the devices disclosed herein can be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, embodiments of the device may be disassembled, and any number of the particular pieces or parts of the device may be selectively replaced or removed in any combination. Upon cleaning and/or replacement of particular parts, embodiments of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various versions in the present disclosure, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus for guiding a biopsy instrument into a breast of a patient supported upon a patient support surface, the apparatus comprising:
(a) a pair of compressive members movably attached to the patient support surface on opposing sides to locate the patient's breast, a selected one of the pair of compressive members further comprising a grid plate having a plurality of grid apertures defined by a plurality of grid walls, the grid plate being accessible from a lateral side and being at least substantially open from a medial side presented to the breast; and
(b) a bracket comprising a first plate and a second plate, wherein the first plate has a slot defined by two side surfaces, wherein the second plate defines a guide opening configured to permit the insertion of a portion of the biopsy instrument through the second plate;
wherein the slot of the first plate is engaged with a grid wall of the grid plate,
wherein the engagement of the grid wall with the slot of the first plate allows the insertion of the biopsy instrument through the guide opening of the second plate and a grid aperture.

2. The apparatus of claim 1, wherein the first plate and the second plate are at a substantially right angle relative to each other.

3. The apparatus of claim 1, further comprising an insert member disposed in the guide opening of the second plate.

4. The apparatus of claim 3, wherein the insert member comprises a flange and a longitudinally extending hollow shaft configured to insertingly receive a portion of the biopsy instrument.

5. The apparatus of claim 1, wherein the second plate has a slot defined by two side surfaces, the slot being configured to receive a selected one of the grid walls.

6. The apparatus of claim 1, wherein the first plate has at least one guide opening configured to permit the insertion of the biopsy instrument through the first plate.

7. The apparatus of claim 1, wherein the two side surfaces of the slot are substantially parallel to each other.

8. The apparatus of claim 1, wherein the second plate defines a plurality of guide openings.

9. The apparatus of claim 1, wherein the bracket it secured to the grid plate by friction between the two side surfaces of the slot and the grid wall.

10. The apparatus of claim 9, wherein the two side surfaces defining the slot include an elastomeric material.

11. The apparatus of claim 1, wherein the bracket is secured to the grid plate by a hinged member that is clipped to the grid wall.

12. An apparatus for guiding a biopsy instrument into a breast of a patient supported upon a patient support surface, the apparatus comprising:
(a) a pair of compressive members movably attached to the patient support surface on opposing sides to locate the patient's breast, a selected one of the pair of compressive members further comprising a grid plate having a plurality of grid apertures defined by a plurality of grid walls, the grid plate being accessible from a lateral side and being at least substantially open from a medial side presented to the breast;
(b) a bracket comprising a first plate and a second plate, wherein the first plate has a slot defined by two side surfaces and one bottom surface, the slot sheathing a grid wall of the grid plate, and wherein the second plate defines a guide opening; and
(c) an insert disposed through the guide opening of the bracket, the insert comprising a flange and a shaft, wherein the shaft defines a lumen configured to permit insertion of the biopsy instrument, and wherein the insert is positioned through at least one guide hole of the second plate.

13. The apparatus of claim 12, wherein the shaft of the insert is generally cylindrical.

14. The apparatus of claim 12, wherein the shaft of the insert is at a non-parallel angle relative to the first plate.

15. A bracket for guiding a biopsy instrument through a grid plate having grid apertures defined by grid walls, the bracket comprising:
(a) a first plate, the first plate having a slot defined by two side surfaces, wherein the slot is configured to insertingly receive a grid wall of the grid plate; and
(b) a second plate coupled with the first plate at an angle, the second plate defining a guide opening configured to permit the insertion of the biopsy instrument through the second plate.
